Europäisches Patentamt

European Patent Office

Office européen des brevets

(19)

(11) Veröffentlichungsnummer : **0 171 800**
**B1**

(12)

# EUROPÄISCHE PATENTSCHRIFT

(45) Veröffentlichungstag der Patentschrift :
11.11.87

(21) Anmeldenummer : 85110182.4

(22) Anmeldetag : 14.08.85

(51) Int. Cl.⁴ : **A 61 F 13/02**, A 61 L 15/03,
A 61 K 9/70, A 61 M 37/00,
B 32 B 33/00, B 32 B 31/00

(54) Pharmako-Heftpflaster und Verfahren zu dessen Herstellung.

(30) Priorität : 17.08.84 DE 3430250
02.04.85 DE 3511963

(43) Veröffentlichungstag der Anmeldung :
19.02.86 Patentblatt 86/08

(45) Bekanntmachung des Hinweises auf die Patenterteilung : 11.11.87 Patentblatt 87/46

(84) Benannte Vertragsstaaten :
AT BE CH DE FR GB IT LI LU NL SE

(56) Entgegenhaltungen :
EP-A- 0 114 125
EP-B- 0 013 606
WO-A-85 /002 87
GB-A- 2 100 605
AUSZÜGE AUS DEN GEBRAUCHSMUSTERN, 21.
Jahrgang, Heft 46, 15. November 1984, München
ALLPACK INDUSTRIELLE LOHNVERPACKUNG
GMBH & CO. KG "Pharmako-Heftpflaster" Seite 2078
(73) Patentinhaber : ALLPACK Industrielle Lohnverpackung GmbH & Co. KG.
Düsseldorfer Strasse 7
D-7050 Waiblingen (DE)

(72) Erfinder : Lauk, Willi
Gerhart-Hauptmann-Strasse 2
D-7060 Schorndorf-Haubersbronn (DE)

(74) Vertreter : Wolff, Michael, Dipl.-Phys.
Kirchheimer Strasse 69
D-7000 Stuttgart 75 (DE)

EP 0 171 800 B1

## Beschreibung

Die Erfindung betrifft ein Pharmako-Heftpflaster mit mindestens zwei vereinigten Teilen, nämlich einem Wirkstoffträger, der eine mit einer flachen Vertiefung zur Arzneimittelaufnahme versehene Verbundfolie, gegebenenfalls mit einer inneren, insbesondere folienförmigen, Schicht aus thermoplastischem Kunststoff, und eine die Vertiefung ringförmig umgebende Randschicht aus druck- und/oder hautwärmeempfindlichem (hautverträglichem) Klebstoff auf der Öffnungsseite der Vertiefung aufweist ; und einer Deckverbundfolie, gegebenenfalls mit einer inneren, insbesondere folienförmigen, Schicht aus thermoplastischem Kunststoff, die mit der Klebstoff-Schicht verbunden ist und die Vertiefung des Wirkstoffträgers bedeckt ; also ein sogenanntes transdermales therapeutisches System.

Bei einem aus der EP-A-0 013 606 bekannten medizinischen Pflaster dieser Art ist das den Wirkstoff enthaltende oder darstellende, in die Haut diffundierende Arzneimittel in einer sogenannten Diffusionsmatrix verteilt, die in die Vertiefung des Wirkstoffträgers eingebettet ist. Die Deckverbundfolie des bekannten Pflasters ist an ihrer Innenseite mit einer der Klebstoff-Schicht entsprechenden sogenannten Ablösungsschicht versehen, von der sich die Klebstoff-Schicht beim Trennen der Deckverbundfolie und des Wirkstoffträgers leicht lösen läßt. Die Diffusionsmatrix ist in die Vertiefung der Wirkstoffträger-Verbundfolie entweder eingesetzt oder eingegossen und darin festgeworden.

Daran ist in erster Linie nachteilig, daß die sogenannte Galenik, also die Aufbereitung des Arzneimittel-Wirkstoffes, von den Eigenschaften der gewählten Matrix abhängt, sodaß bei der Galenik auf diese Eigenschaften Rücksicht genommen werden muß, was eine Optimierung der Galenik erschwert.

Daher liegt der Erfindung zunächst die Aufgabe zugrunde, ein diesen ersten Nachteil vermeidendes Pharmako-Heftpflaster zu schaffen, das eine Optimierung der Galenik unabhängig von Matrixeigenschaften ermöglicht.

Diese Aufgabe ist bei einem Pflaster der eingangs genannten Art erfindungsgemäß dadurch gelöst, daß die Vertiefung des Wirkstoffträgers mittels einer für den Wirkstoff durchlässigen, mit der Verbundfolie des Wirkstoffträgers fest verbundenen Membran verschlossen ist ; und daß die Deckverbundfolie längs eines zwischen der Membran und der Klebstoff-Schicht verlaufenden Ringes hermetisch mit der Verbundfolie des Wirkstoffträgers verbunden ist.

Aus der EP-A-0 114 125 ist ein Applikations-System bekannt, bei dem die Vertiefung des Wirkstoffträgers mittels einer mikroporösen, mit dem Wirkstoffträger fest verbundenen Membran verschlossen ist und bei dem im Ringraum zwischen der Membran und einer äußeren Klebstoff-Schicht an der Deckverbundfolie ein die Vertiefung umlaufender Ring anliegt. Dieser Ring ist jedoch nicht mit der Deckverbundfolie hermetisch verbunden.

Weiterhin ist in der WO 85/00287 (Stand der Technik gemäß Art. 54(3) EPÜ hinsichtlich des Erzeugnisses (Pharmako-Heftpflaster) für alle Benennungsstaaten mit Ausnahme von Italien) ein Pflaster zum Aufbringen auf die Haut offenbart. Dieses Pflaster weist ebenfalls zwischen einer Membran und der Klebstoff-Schicht keine hermetische Verbindung in Form eines die Vertiefung zur Arzneimittelaufnahme umlaufenden Ringes auf.

Vorteilhafterweise wird bei der Erfindung erreicht, daß die Galenik völlig frei gewählt und danach eine dazu passende Membran ausgesucht werden kann, welche dazu dient, das Arzneimittel in der Vertiefung des Wirkstoffträgers zurückzuhalten und allmählich an die Haut abzugeben.

Bei dem bekannten Pflaster, das bis zum Gebrauch in einem hermetisch abgeschlossenen Flachbeutel liegt, ist in zweiter Linie nachteilig, daß die Diffusionsmatrix das in ihr enthaltene Arzneimittel, gegebenenfalls den Wirkstoff selbst, nicht vollständig an die Haut abzugeben vermag, insbesondere nicht den am Grund der Vertiefung befindlichen Teil des Arzneimittels, weil dieses selbst erst durch die Matrix diffundieren muß, bevor es in die damit in Berührung stehende Haut hineindiffundieren kann. Infolgedessen ist die Dosierung unzureichend, wenn nicht von vornherein entsprechend mehr Arzneimittel in der Matrix enthalten ist, was die Gestehungskosten unter Umständen erheblich erhöht. Der Gebrauch des bekannten Pflasters kann also zur Fehldosierung des applizierten Arzneimittels führen.

Der Erfindung liegt daher ferner die Aufgabe zugrunde, ein diesen Nachteil vermeidendes Pharmako-Heftpflaster zu schaffen, welches durch angenähert restlose Erschöpfung des Arzneimittels zu dessen fast richtiger Dosierung führt.

Diese Aufgabe ist bei einem Pflaster der eingangs genannten Art erfindungsgemäß dadurch gelöst, daß das den Wirkstoff enthaltende oder darstellende, in die Haut diffundierende Arzneimittel die Vertiefung des Wirkstoffträgers allein ausfüllt, welche am besten mittels der für den Wirkstoff durchlässigen, mit der Verbundfolie des Wirkstoffträgers fest verbundenen Membran verschlossen ist, wobei die Deckverbundfolie längs des zwischen der Membran und der Klebstoff-Schicht verlaufenden Ringes hermetisch mit der Verbundfolie des Wirkstoffträgers verbunden ist.

« Hermetisch » bedeutet, daß weder Luft in das Zentrum des Pflasters eindringen noch Wirkstoff aus dem Zentrum heraustreten kann.

Dadurch wird vorteilhafterweise erreicht, daß das Arzneimittel, das von der semipermeablen oder beispielsweise durch Perforation diffusionsfähig gemachten Membran zwar en masse zurückgehalten, jedoch andauernd in kleinen Mengen an die Haut abgegeben wird, nahezu vollkommen verbraucht werden kann, ohne daß nennenswerte Rückstände im Wirkstoffträger verbleiben. Damit ist die Dosierung des Arzneimittels erheblich erleichtert und überdies wirtschaftlich durchführbar, weil nicht für einen Überschuß gesorgt zu werden braucht, wenn eine Mindestdosierung verlangt wird. Darüberhinaus entfällt eine besondere Hülle für das Pflaster, da dessen Teile nicht nur verklebt, sondern auch verschweißt sind.

Es ist in der DE-U-8 424 387 (ALLPACK) (Prioritätsgrundlage für vorliegende Erzeugnisansprüche) bereits vorgeschlagen worden, die letztere Aufgabe bei einem Pharmako-Heftpflaster der eingangs genannten Art dadurch zu lösen, daß das den Wirkstoff enthaltende oder darstellende, in die Haut diffundierende Arzneimittel die Vertiefung des Wirkstoffträgers allein ausfüllt, welche mittels einer für den Wirkstoff durchlässigen, mit der Verbundfolie des Wirkstoffträgers fest verbundenen Membran verschlossen ist; und daß die Deckverbundfolie längs eines zwischen der Membran und der Klebstoff-Schicht verlaufenden Ringes hermetisch mit der Verbundfolie des Wirkstoffträgers verbunden ist und gegebenenfalls unmittelbar mit ihrer ebenen Fläche an der Klebstoffschicht anliegt. In diesem Fall unterscheidet sich eine bevorzugte Ausführungsform des erfindungsgemäßen Pflasters von dem früher vorgeschlagenen Pflaster dadurch, daß die Deckverbundfolie definitiv mittelbar mit ihrer ebenen Innenfläche an der Klebstoff-Schicht anliegt, wobei diese einem die Wirkstoffträger-Vertiefung ringförmig umgebenden dritten Pflasterteil angehört, der einerseits mit der Wirkstoffträger-Verbundfolie und andererseits mit der Deckverbundfolie fest verbunden ist sowie an der dieser zugewandten Haftfläche der Klebstoff-Schicht in zwei Unterteile trennbar ist, deren Trennung diese Haftfläche freilegt.

Durch diesen nach dem Wirkstoffträger und der Deckverbundfolie dritten Pflasterteil neben der Membran wird zusätzlich erreicht, daß die zur Ablösung der Deckverbundfolie vom Wirkstoffträger und damit zur Freilegung der Klebstoff-Schicht sowie der Membran am besten geeigneten Materialbedingungen am leichtesten erfüllt werden können, weil die beiden Unterteile des dritten Pflasterteiles, von denen einer die Klebstoff-Schicht und der andere eine Gegenschicht aufweist, selbst mehrschichtig aufgebaut sein können, wobei die für den paarweisen Schichtverbund am besten geeigneten Materialkombinationen, auch an den Grenzen zwischen dem dritten Pflasterteil einerseits und der Wirkstoffträger-Verbundfolie bzw. der Deckverbundfolie andererseits, auswählbar sind.

Bei der bevorzugten Ausführungsform des erfindungsgemäßen Pflasters besteht die Membran wie der dritte Pflasterteil aus wenigstens drei Schichten, von denen eine als Klebstoff-Schicht zwischen den beiden anderen Schichten angeordnet ist. Dadurch ist die mit der Haut in Berührung zu bringende Haftfläche der Klebstoff-Schicht vor Abtrennung des anhaftenden Unterteiles des dritten Pflasterteiles jederzeit vor unerwünschtem Kontakt geschützt.

Die bevorzugte Ausführungsform zeichnet sich dadurch aus, daß die Membran und der dritte Pflasterteil je aus einer mit einer thermoplastischen Kunststoff-Schicht der Wirkstoffträger-Verbundfolie verschweißten thermoplastischen Kunststoff-Schicht, aus der auf diese Schicht aufgebrachten Klebstoff-Schicht, aus einer mit einer thermoplastischen Kunststoff-Schicht der Deckverbundfolie verschweißten thermoplastischen Kunststoff-Schicht und aus einer auf diese Schicht aufgebrachten Schicht, vorzugsweise aus Silikon, bestehen, die an der Klebstoff-Schicht anliegt, wobei diese Schicht sowie die mit der Wirkstoffträger-Verbundfolie verbundene Kunststoff-Schicht der Membran für den Wirkstoff durchlässig sind und gegebenenfalls die Silikon-Schicht und/oder die mit der Deckverbundfolie verbundene Kunststoff-Schicht der Membran für den Wirkstoff undurchlässig ist bzw. sind. Einer dieser alternativen Fälle müßte gegeben sein, wenn die Deckverbundfolie selbst für den Wirkstoff durchlässig wäre, was unzweckmäßig erscheint. Daß Membran und dritter Pflasterteil die gleiche Schichtenfolge aufweisen, ist bei der Herstellung und beim Gebrauch des Pflasters von Vorteil. Ein vierschichtiger Aufbau von Membran und drittem Pflasterteil erscheint zweckmäßig und hinreichend.

Bei der bevorzugten Ausführungsform weist die Wirkstoffträger-Verbundfolie eine ihre Vertiefung umringende, außerhalb der Membran und innerhalb des dritten Pflasterteiles gelegene Erhebung und/oder die Deckverbundfolie eine entsprechende Erhebung auf, deren Höhe einzeln bzw. gemeinsam der übereinstimmenden Dicke des dritten Pflasterteiles und der Membran entspricht. Infolgedessen erfährt die Deckverbundfolie beim Verschweißen derselben mit der Wirkstoffträger-Verbundfolie zum hermetischen Verbinden beider längs des Ringes zwischen Membran und konzentrischem drittem Pflasterteil keine wesentliche Verformung, sodaß Außenaufschriften, wie Applikationshinweise, auch im Bereich des genannten Ringes gut lesbar bleiben.

In anderer Hinsicht vorteilhaft erscheint eine Ausführungsform des erfindungsgemäßen Pharmako-Heftpflasters, bei welcher die Membran und der dritte Pflasterteil einstückig ausgebildet sind, sodaß eine Erhebung der Wirkstoffträger-Verbundfolie zur Anlage an der anfangs eventuell ebenen Deckverbundfolie bzw. an der Wirkstoffträger-Verbundfolie entfallen kann. Die einstückige Ausbildung erlaubt nämlich nicht nur eine rationelle Pflasterfertigung, sondern auch ein garantiert müheloses Trennen der Deckverbundfolie vom Wirkstoffträger, weil dabei die aufzureißende Verschweißung der Wirkstoffträger-Verbundfolie mit der Deckverbundfolie über die Erhebung/en entfällt, und mag der Ring der hermetischen Verbindung noch so schmal sein.

Bei der bevorzugten Ausführungsform ist von der Deckverbundfolie und dem fest mit dieser verbundenen Unterteil des dritten Pflasterteiles mittels einer Trennfuge ein Randstück abgeteilt, welches im Flächenbereich der Klebstoff-Schicht des anderen Unterteiles des dritten Pflasterteiles liegt und an dieser haftet. Durch Festhalten des Pflasters mit beiden Händen diesseits bzw. jenseits der Trennfuge und Biegen des Pflasters um die Trennfuge kann die Klebstoff-Schicht im Bereich der Trennfuge bequem freigelegt werden. Ist aber erst einmal der Anfang gemacht, dann läßt sich die Deckverbundfolie mit anhängendem Unterteil des dritten Pflasterteiles restlos von dessen anderem Unterteil trennen, um die Haftfläche der Klebstoff-Schicht freizulegen.

Bei einer anderen Ausführungsform des erfindungsgemäßen Pharmako-Heftpflasters, dessen Wirkstoffträger-Verbundfolie wie im Falle des bekannten Pflasters definitiv eine der Deckverbundfolie zugewandte Schicht aus thermoplastischem Kunststoff aufweist, ist vorgesehen, daß die semipermeable Membran aus dem Verbund einer mit der Kunststoff-Schicht der Wirkstoffträger-Verbundfolie verschweißten porösen thermoplastischen Kunststoff-Schicht und einer Vliesschicht besteht, welche glyzeringetränkt wird und eine Hautanlagefläche bildet. Solche Membranen sind für die Ultrafiltration käuflich und es muß nur die für eine effektive Diffusion des Wirkstoffes geeignetste ausgesucht werden. Die Glyzerinträkung ist nicht unbedingt erforderlich.

Bei der anderen Ausführungsform, deren Verbundfolien wie im Falle des bekannten Pflasters an ihren einander zugewandten Seiten definitiv je eine thermoplastische Kunststoff-Folie aufweisen, ist durch eine Verschweißung beider Folien für die hermetische Verbindung der Deckverbundfolie mit dem Wirkstoffträger gesorgt. Die Technik des Heißversiegelns ist allgemein bekannt und ohne Schwierigkeiten durchführbar. Eine der Kunststoff-Folien kann durch eine Siegellackschicht ersetzt sein, die sich eventuell besser mit der anderen Folie verbindet.

Bei der anderen Ausführungsform ist von der Deckverbundfolie mittels einer Trennfuge ein Randstück abgeteilt, welches im Flächenbereich der Klebstoff-Schicht liegt und vollständig an dieser haftet, sodaß das Pflaster im Bereich dieses Randstückes von Daumen und Zeigefinger einer Hand erfaßt und die Kante der Deckverbundfolie an der Trennfuge mit einem Fingernagel der anderen Hand untergriffen werden kann, worauf der größte Teil der Deckverbundfolie mit dieser anderen Hand vom Wirkstoffträger abgezogen werden kann, auf dem dann nur das Randstück verbleibt, das nicht unbedingt vom Wirkstoffträger entfernt werden muß, weil ein Teil der Klebstoff-Schicht auch zwischen Randstück und Vertiefung der Wirkstoffträger-Verbundfolie wirksam ist.

Um ein eventuelles Austrocknen der Klebstoff-Schicht durch Verdunsten des Lösungsmittels zu verhindern, kann die Deckverbundfolie längs eines außerhalb der Klebstoff-Schicht am Pflasterrand verlaufenden Ringes hermetisch mit der Wirkstoffträger-Verbundfolie verbunden werden. Gegebenenfalls können die Deckverbundfolie statt der zuvor genannten Einrichtung zur Pflasteröffnung, und die Wirkstoffträger-Verbundfolie außerhalb des äußeren Ringes einer hermetischen Verbindung beider Folien je einen von zwei sich vorzugsweise gegenseitig deckenden, vorspringenden Aufreißlappen bilden, die bei einem kreisscheibenförmigen Pflaster dreieckig gestaltet sind. Anhand der beiden Lappen kann die hermetische, d. h. hier lösungsmitteldichte, Folienverbindung am Pflasterrand leicht aufgerissen werden.

Die Erfindung betrifft auch ein Verfahren zum Herstellen des erfindungsgemäßen Pharmako-Heftpflasters, bei welchem wie im Falle der EP-A-0 013 606 zwei separat erzeugte äußere Pflasterteile mittels einer inneren Klebstoff-Schicht trennbar verbunden sind.

Aus der DE-U-8 424 387 (Stand der Technik gemäß Art. 54 (1) (2) EPÜ für die Verfahrensansprüche) ist zusätzlich bekannt, ein derartiges Pflaster mit einer Membran als Abdeckung für die Vertiefung des Wirkstoffträgers und mit einem Ring zwischen der Membran und der Klebstoff-Schicht zur hermetischen Verbindung der Deckverbundfolie mit der Verbundfolie des Wirkstoffträgers herzustellen.

Darüberhinaus ist in der WO 85/00287 (Stand der Technik gemäß Art. 54 (1) (2) EPÜ für die Verfahrensansprüche) offenbart, daß die Klebstoffschicht als Bestandteil eines inneren, dritten Pflasterteiles erzeugt wird, der separat von den beiden äußeren Pflasterteilen erzeugt und danach mit einem der beiden fest verbunden wird.

Der Erfindung die Aufgabe zugrunde, ein solches Verfahren anzugeben, das eine rationelle Massenfertigung erfindungsgemäßer Pflaster ermöglicht.

Diese Aufgabe ist erfindungsgemäß dadurch gelöst, daß die Klebstoff-Schicht als Bestandteil eines inneren, dritten Pflasterteiles erzeugt wird, der separat von den beiden äußeren Pflasterteilen erzeugt und danach mit diesen fest verbunden wird.

Dadurch wird vorteilhafterweise erreicht, daß zwar wie bei dem bekannten Verfahren nach der EP-A-0 013 606 die Klebstoff-Schicht als Bestandteil eines Pflasterteiles erzeugt werden muß, jedoch nicht des Wirkstoffträgers und als Beschichtung von dessen Verbundfolie wie bei dem bekannten Verfahren, sondern als Bestandteil eines unabhängig von den beiden anderen Pflasterteilen (ungeachtet einer Membran) erzeugbaren dritten Pflasterteiles, der die Klebstoff-Schicht enthält und deshalb ohne Haftprobleme zwischen die beiden anderen Pflasterteile eingebracht sowie in seinem Zentrum mit einer Membran versehen werden kann, falls diese nicht ohnehin das Zentrum des dritten Pflasterteiles bildet.

Bei der bevorzugten Durchführungsweise des erfindungsgemäßen Verfahrens zum Herstellen der bevorzugten Ausführungsform des erfindungsgemäßen Pharmako-Heftpflasters werden dessen Membran und sein dritter Pflasterteil gleichzeitig erzeugt, sodaß die Zahl der diesbezüglichen Verfahrensschritte nur halb so groß ist und dementsprechend die Produktionszeit für Membran und dritten Pflasterteil halbiert wird. Das gilt auch dann, wenn die Membran beispielsweise mittels Korpuskularstrahlung perforiert wird, denn der dritte Pflasterteil kann ohne schädliche Wirkung auf die Pflasterfunktion mitperforiert werden.

Bei der bevorzugten Durchführungsweise werden die Membran und der dritte Pflasterteil in einem einzigen Stück gefertigt, aus dem ein der Erhebung der Wirkstoffträger-Verbundfolie entsprechender Ring herausgetrennt wird, der Membran und dritten Pflasterteil vereinzelt und dem Ring Platz macht, längs dessen die Deckverbundfolie hermetisch mit der Wirkstoffträger-Verbundfolie verbunden wird, um die das Arzneimittel in der Wirkstoffträger-Vertiefung bedeckende Membran zu versiegeln.

Bei der bevorzugten Durchführungsweise wird das Verschweißen der Membran, der Erhebung der Wirkstoffträger-Verbundfolie und des dritten Pflasterteiles einerseits mit der Deckverbundfolie andererseits gleichzeitig durchgeführt, damit an Produktionszeit gespart wird. Dazu wird bei der bevorzugten Durchführungsweise auch das Verschweißen der Membran und des dritten Pflasterteiles einerseits mit der Wirkstoffträger-Verbundfolie andererseits gleichzeitig durchgeführt, und zwar insbesondere so, daß beide Verschweißvorgänge an der Deckverbundfolie bzw. der Wirkstoffträger-Verbundfolie simultan erfolgen.

Im folgenden ist die Erfindung anhand zweier durch die Zeichnung beispielhaft dargestellter Ausführungsformen des erfindungsgemäßen Pharmako-Heftpflasters im einzelnen erläutert. Es zeigt:

Fig. 1 eine Draufsicht auf die erste Ausführungsform unter Ansicht ihrer Deckverbundfolie;

Fig. 2 ein vergrößert und in Höhenrichtung gedehnt dargestelltes Bild eines zentralen Schnittes durch die erste Ausführungsform vor der Vereinigung ihres unteren Wirkstoffträgers mit der oberen Deckverbundfolie;

Fig. 3 ein ebenso vergrößertes, aber weniger gedehntes Bild desselben Schnittes nach der Linie III-III in Fig. 1 durch die erste Ausführungsform nach der Vereinigung von Wirkstoffträger und Deckverbundfolie; und

Fig. 4 ein vergrößert und in Höhenrichtung gedehnt dargestelltes Bild eines zentralen Schnittes durch die zweite Ausführungsform nach Vereinigung aller Pflasterteile, schematisch.

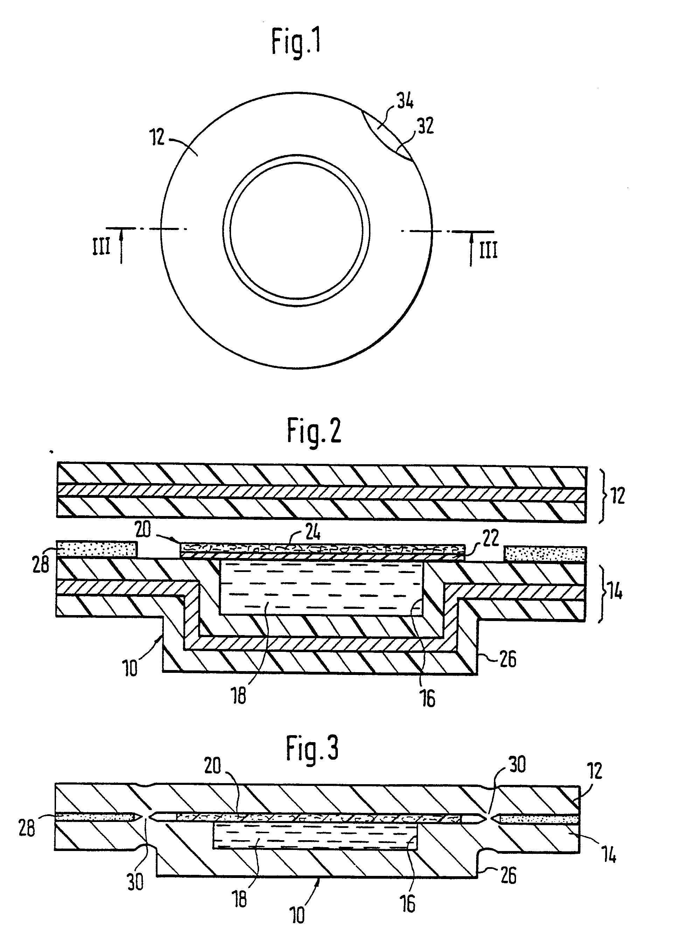

Im ersten Ausführungsbeispiel besteht das erfindungsgemäße Pharmako-Heftpflaster gemäß Fig. 1 bis 3 aus zwei vereinigten Teilen, nämlich einem Wirkstoffträger 10 und einer Deckverbundfolie 12. Der Wirkstoffträger 10 weist eine seine Außenseite bildende Verbundfolie 14 auf, die in ihrer Schichtenfolge dem Aufbau der ebenen Deckverbundfolie 12 entspricht, die aus je einer ihre Außen- und Innenseite bildenden Polyamid- bzw. thermoplastischen Polyvinylchlorid (PVC)-Folie und aus einer dazwischen angeordneten Aluminium-Folie besteht. Sowohl der Wirkstoffträger 10 als auch die Deckverbundfolie 12 besitzen gemäß Fig. 1 einen kreisrunden Grundriß, und zwar denselben, sodaß sie sich gegenseitig völlig decken.

Der Wirkstoffträger 10 ist in seinem Zentrum mit einer beispielsweise durch Tiefziehen der noch ebenen Verbundfolie 14 entstandenen zentralen, kreiszylindrischen Vertiefung 16 versehen, in die ein flüssiges oder pastoses Arzneimittel 18 eingebettet ist, das die Vertiefung bis zu ihrem Rand ausfüllt, auf dem eine ebene, kreisrunde, semipermeable Membran des Wirkstoffträgers 10 liegt, welche aus einer mit der anliegenden PVC-Folie der Verbundfolie 14 thermisch verschweißten Kunststoff-Schicht 22 und aus einer mit dieser im Verbund stehenden Vliesschicht 24 besteht, die der Deckverbundfolie 12 zugewandt ist. Dabei ist die Anordnung so getroffen, daß die Membran 20 die Vertiefung 16 zwar an deren Öffnungsseite vollständig schließt, jedoch einen Außendurchmesser aufweist, der kleiner ist als der Außendurchmesser des durch die Vertiefung der Verbundfolie 14 entstandenen kreiszylindrischen Vorsprungs 26 des Wirkstoffträgers 10 nach außen.

Die Verbundfolie 14 des Wirkstoffträgers 10 ist konzentrisch zu dessen Membran 20 mit einer kreisringförmigen Klebstoff-Schicht 28 belegt, deren Außendurchmesser dem Außendurchmesser der vertieften Verbundfolie und deren Stärke der Dicke der Membran 20 ungefähr entspricht. Der Innendurchmesser der Klebstoff-Schicht 28 ist größer als der Außendurchmesser des Vorsprungs 26 und damit wesentlich größer als der Außendurchmesser der Membran 20. Als Klebstoff-Schicht 28 kommt außer einer klebrigen Schaumschicht ein beidseitig klebendes Ringband infrage.

Zur Vereinigung der Deckverbundfolie 12 mit dem Wirkstoffträger 10 wird die flache Deckverbundfolie 12 so auf den topfähnlichen Wirkstoffträger 10 gelegt, daß sie sich genau decken. Anschließend werden beide längs einer kreisringförmigen Zone 30, die radial gesehen zwischen der Klebstoff-Schicht 28 und dem Vorsprung 26 liegt, derart thermisch verschweißt, daß die aneinander-gelegten PVC-Schichten (Laminate) der Deckverbundfolie 12 einerseits bzw. der Verbundfolie 14 des Wirkstoffträgers 10 andererseits verschmelzen. Durch diese Heißversiegelung entsteht eine hermetische Verbindung der Deckverbundfolie 12 und des Wirkstoffträgers 10.

Zum Trennen der Deckverbundfolie 12 vom Wirkstoffträger 10 weist die Deckverbundfolie ein mittels einer kreisbogenförmigen Trennfuge 32 abgeteiltes, bikonvexes Randstück 34 auf.

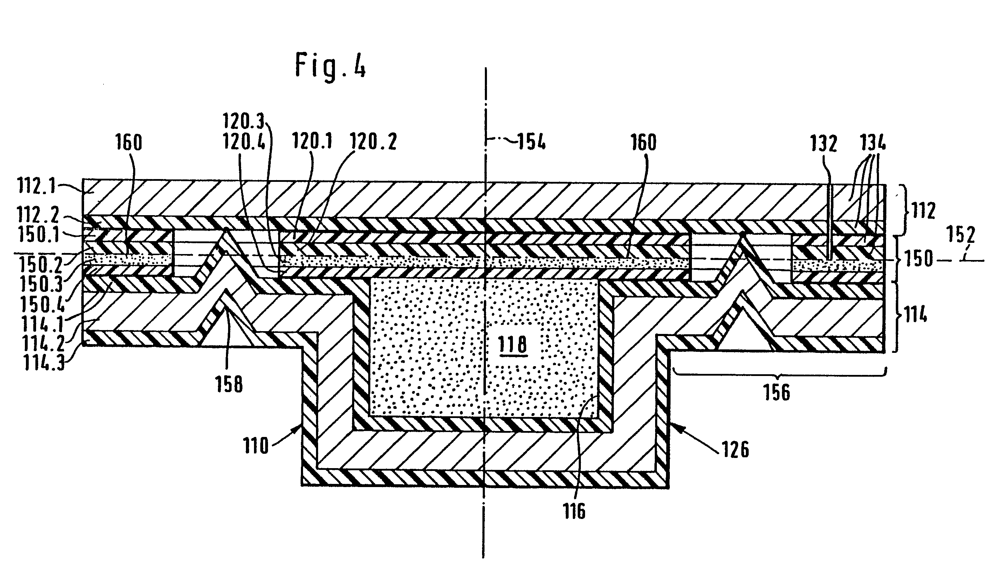

Im bevorzugten zweiten Ausführungsbeispiel besteht das erfindungsgemäße Pharmako-Heftpflaster ohne das Arzneimittel gemäß Fig. 4 aus vier vereinigten Teilen, nämlich einem Wirkstoffträger 110, einer Deckverbundfolie 112, einem Pflasterring 150 als drittem Pflasterteil und einer Membran 120. Der Außenumfang dieser Pflasterteile und falls vorhanden auch deren Innenumfang sind kreisförmig, wobei alle Kreismittelpunkte auf der Rotationssymmetrieachse 154 liegen.

Der aus einer Verbundfolie 114 gefertigte Wirkstoffträger 110 besitzt eine zentrale konische oder im Ausführungsbeispiel zylindrische Vertiefung 116, in die ein flüssiges oder pastoses Arzneimittel 118 eingebettet ist, das die Vertiefung bis zu ihrem Rand ausfüllt, und welcher ein zentraler konischer bzw. zylindrischer Vorsprung 126 des Wirkstoffträgers nach außen entspricht, und weist axial von innen nach außen eine innere thermoplastische Kunststoff-Schicht 114.1 aus Polyäthylen, eine mittlere Metallfolie 114.2 aus Aluminium und eine äußere thermoplastische Kunststoff-Schicht 114.3 aus Polyamid auf, die je einige Zehntel Millimeter dick sind. Der den Vorsprung 126 säumende ringförmige Rand 156 des Wirkstoffträgers 110 liegt in einer Ebene, auf der die Achse 154 senkrecht steht, und weist eine konzentrische sickenartige Erhebung 158 auf, die gegen die Deckverbundfolie 112 vorspringt und diese berührt sowie in den konzentrischen Ringraum zwischen Membran 120 und Pflasterring 150 eingreift. Diese Erhebung könnte ersatzweise oder zusätzlich an der Deckverbundfolie 112 vorgesehen sein ; falls zusätzlich, würden sich beide Erhebungen auf halbem Wege treffen.

Die völlig ebene Deckverbundfolie 112 weist axial von außen nach innen eine äußere Metallfolie 112.1 aus Aluminium und eine innere thermoplastische Kunststoff-Schicht 112.2 aus einem Ionomer auf.

Die völlig ebene Membran 120 und der gleichfalls ebene Plasterring 150 weisen übereinstimmend eine mit der Schicht 112.2 der Deckverbundfolie 112 verschweißte thermoplastische Kunststoff-Schicht 120.1 bzw. 150.1 in Form eines Aufstriches, eine mit diesem verbundene Silikon-Schicht 120.2 bzw. 150.2, eine von der Silikon-Schicht leicht lösbare Klebstoff-Schicht 120.3 bzw. 150.3 (Randschicht) und eine mit der Schicht 114.1 der Wirkstoffträger-Verbundfolie 114 thermisch verschweißte thermoplastische Kunststoff-Schicht 120.4 bzw. 150.4 aus Polyäthylen auf, welche die Klebstoff-Schicht trägt. Während die Schichten 120.1 und 150.1 sowie 150.4 über ihre ganze axial äußere Oberfläche mit der Schicht 112.2 bzw. 114.1 verschweißt sind, und zwar so, daß die Deckverbundfolie 112 die Wirkstoffträger-Verbundfolie 114 und der axial dazwischen angeordnete Pflasterring 150 mit ihren radial äußeren Umfängen bündig liegen, ist die Schicht 120.4 nur an ihrem Rand, der die Vertiefung 116 säumt und das Arzneimittel 118 nicht berührt, mit der Schicht 114.1 verschweißt. Thermisch verschweißt ist auch die Erhebung 158 des Wirkstoffträgers 110, und zwar mit der Schicht 112.2 der Deckverbundfolie 112, längs eines Kreisringes um die Achse 154. Die Schichten 120.3 (Klebstoff) und 120.4 (Polyäthylen) sind für den im Arzneimittel 118 enthaltenen Wirkstoff durchlässig.

Zum Applizieren des anhand Fig. 4 dargestellten Pharmako-Heftpflasters auf der Haut ist dieses in der Trennebene 152, auf der die Achse 154 senkrecht steht, zu Öffnen, indem die Deckverbundfolie 112 mit den Schichten 120.1 und 120.2 der Membran 120 sowie den Schichten 150.1 und 150.2 des Pflasterringes 150 von einer Stelle des Pflasterumfanges her von der Wirkstoffträger-Verbundfolie 114 mit den Schichten 120.3 und 120.4 der Membran 120 sowie den Schichten 150.3 und 150.4 des Pflasterringes 150 abgezogen wird, wobei sich die Silikon-Schichten 120.2 und 150.2 von den Klebstoff-Schichten 120.3 und 150.3 leicht lösen, welche anschließend auf die Haut gedrückt werden, wobei die Erhebung 158 des Wirkstoffträgers 110 praktisch nicht stört, weil das Absolutmaß des axialen Überstehens der Erhebung über die Trennebene 152 größenordnungsmäßig nur einige Zehntel Milimeter beträgt, um welche die Haut nachgibt. Erforderlichenfalls kann der Wirkstoffträger mit einer Erhebung versehen werden, die nur bis zur Trennebene 152 reicht, wo diese Erhebung auf eine entsprechende Vertiefung der dann nicht mehr ebenen Deckverbundfolie trifft.

Um die Pflasteröffnung anfangs zu erleichtern, sind die Deckverbundfolie 112 und die Schichten 150.1 sowie 150.2 mit einer gemeinsamen Trennfuge 132 versehen, die ein Randstück 134 abteilt, das an der Klebstoff-Schicht 150.3 des Pflasterringes 150 haftet, der aus einem mit der Deckverbundfolie 112 verbundenen Unterteil mit den Schichten 150.1 und 150.2 sowie aus einem mit der Wirkstoffträger-Verbundfolie 114 verbundenen Unterteil mit den Schichten 150.3 und 150.4 besteht, von denen die Klebstoff-Schicht 150.3 mit ihrer Haftfläche 160 an der Schicht 150.2 anliegt.

Diese Haftfläche 160 setzt sich radial innen an der Membran-Schicht 120.3 fort.

### Patentansprüche

1. Pharmako-Heftpflaster mit zwei vereinigten Teilen, nämlich einem Wirkstoffträger (10 ; 110), der eine mit einer flachen Vertiefung (16 ; 116) zur Arzneimittelaufnahme versehene Verbundfolie (14 ; 114), gegebenenfalls (Fig. 4) mit einer inneren, insbesondere folienförmigen, Schicht (114.1) aus thermoplastischem Kunststoff, und eine die Vertiefung (16 ; 116) ringförmig umgebende Randschicht (28 ; 150.3) aus druck- und/oder hautwärmeempfindlichem Klebstoff auf der Öffnungsseite der Vertiefung (16 ; 116) aufweist ; und einer Deckverbundfolie (12 ; 112), gegebenenfalls (Fig. 4) mit einer inneren, insbesondere folienförmigen, Schicht (112.2) aus thermoplastischem Kunststoff, die mit der Klebstoff-Schicht (28 ; 150.3) verbunden ist und die Vertiefung (16 ; 116) des Wirkstoffträgers (10 ; 110) bedeckt, dadurch gekennzeichnet, daß die Vertiefung (16 ; 116) des Wirkstoffträgers (10 ; 110) mittels einer für den Wirkstoff durchlässigen, mit der Verbundfolie (14 ; 114) des Wirkstoffträgers (10 ; 110) fest verbundenen Membran (20 ; 120) verschlossen ist ; und daß die Deckverbundfolie (12 ; 112) längs eines zwischen der Membran (20 ; 120) und der Klebstoff-Schicht (28 ; 150.3) verlaufenden Ringes (30 ; 158) hermetisch mit der Verbundfolie (14 ; 114) des Wirkstoffträgers (10 ; 110) verbunden ist.

2. Pharmako-Heftpflaster nach Anspruch 1, dadurch gekennzeichnet, daß das den Wirkstoff enthaltende oder darstellende, in die Haut diffundierende Arzneimittel (18 ; 118) die Vertiefung (16 ; 116) des Wirkstoffträgers (10 ; 110) allein ausfüllt.

3. Pharmako-Heftpflaster nach Anspruch 1 oder 2, dadurch gekennzeichnet, daß die Deckverbundfolie (112) mittelbar mit ihrer ebenen Innenfläche an der Klebstoff-Schicht (150.3) anliegt, wobei diese einem die Vertiefung (116) ringförmig umgebenden dritten Teil (150) angehört, der einerseits mit der Verbundfolie (114) des Wirkstoffträgers (110) und andererseits mit der Deckverbundfolie (112) fest verbunden ist sowie an der dieser zugewandten Haftfläche (160) der Klebstoff-Schicht (150.3) in zwei Teile (150.1, 150.2 ; 150.3, 150.4) trennbar ist, deren Trennung diese Haftfläche freilegt.

4. Pharmako-Heftpflaster nach einem der Ansprüche 1 bis 3, dadurch gekennzeichnet, daß die Membran (120) ebenso wie der dritte Pflasterteil (150) aus wenigstens drei Schichten (120.1 bis 120.4) besteht, von denen eine als Klebstoff-Schicht (120.3) zwischen den beiden anderen Schichten (120.2, 120.4) angeordnet ist, und daß die Membran (120) gegebenenfalls zuzammen mit dem dritten Pflasterteil (150) aus einem Stück geformt sind.

5. Pharmako-Heftpflaster nach Anspruch 4, dadurch gekennzeichnet, daß die Membran (120) und der dritte Pflasterteil (150) je aus einer mit einer thermoplastischen Kunststoff-Schicht (114.1) der Wirkstoffträger (110)-Verbundfolie (114) verschweißten thermoplastischen Kunststoff-Schicht (120.4 bzw. 150.4), aus der auf diese Schicht (120.4 bzw. 150.4) aufgebrachten Klebstoff-Schicht (120.3 bzw. 150.3), aus einer mit einer thermoplastischen Kunststoff-Schicht (112.2) der Deckverbundfolie (112) verschweißten thermoplastischen Kunststoff-Schicht (120.1 bzw. 150.1) und aus einer auf diese Schicht (120.1 bzw. 150.1) aufgebrachten Schicht (120.2 bzw. 150.2) vorzugsweise aus Silikon bestehen, die an der Klebstoff-Schicht (120.3 bzw. 150.3) anliegt, wobei die Klebstoff-Schicht (120.3 bzw. 150.3) sowie die mit der Wirkstoffträger-Verbundfolie (114) verbundene Kunststoff-Schicht (120.4) der Membran (120) für den Wirkstoff durchlässig sind und gegebenenfalls die Silikon-Schicht (120.2) und/oder die mit der Deckverbundfolie (112) verbundene Kunststoff-Schicht (120.1) der Membran (120) für den Wirkstoff undurchlässig ist.

6. Pharmako-Heftpflaster nach einem der Ansprüche 1 bis 5, dadurch gekennzeichnet, daß die Wirkstoffträger (110)-Verbundfolie (114) eine ihre Vertiefung (116) umringende, außerhalb der Membran (120) und innerhalb des dritten Pflasterteiles (150) gelegene Erhebung (158) und/oder die Deckverbundfolie (112) eine entsprechende Erhebung aufweist, deren Höhe einzeln bzw. gemeinsam der übereinstimmenden Dicke des dritten Pflasterteiles (150) und der Membran (120) entspricht.

7. Verfahren zum Herstellen des Pharmako-Heftpflasters nach Anspruch 3, bei welchem zwei separat erzeugte äußere Pflasterteile (110 und 112) mittels einer inneren Klebstoff-Schicht (150.3) trennbar verbunden sind, dadurch gekennzeichnet, daß die Klebstoff-Schicht (150.3) als Bestandteil eines inneren, dritten Pflasterteiles (150) erzeugt wird, der separat von den beiden äußeren Pflasterteilen (110 und 112) erzeugt und danach mit diesen fest verbunden wird.

8. Verfahren nach Anspruch 7, zum Herstellen des Pharmako-Heftpflasters nach Anspruch 4, dadurch gekennzeichnet, daß die Membran (120) und der dritte Pflasterteil (150) gleichzeitig erzeugt werden.

9. Verfahren nach Anspruch 8, zum Herstellen des Pharmako-Heftpflasters nach Anspruch 6, dadurch gekennzeichnet, daß die Membran (120) und der dritte Pflasterteil (150) in einem einzigen Stück gefertigt werden, aus dem ein der Erhebung (158) der Wirkstoffträger (110)-Verbundfolie (114) entsprechender Ring herausgetrennt wird, der die Membran (120) und den dritten Pflasterteil (150) vereinzelt.

10. Verfahren nach einem der Ansprüche 7 bis 9, zum Herstellen des Pharmako-Heftpflasters nach Anspruch 6 mit 5 dadurch gekennzeichnet, daß das Verschweißen der Membran (120), der Erhebung (158) der Wirkstoffträger (110)-Verbundfolie (114) und des dritten Pflasterteiles (150) einerseits mit der Deckverbundfolie (112) andererseits gleichzeitig durchgeführt wird.

11. Verfahren nach einem der Ansprüche 7 bis 9 oder insbesondere Anspruch 10, zum Herstellen des Pharmako-Heftpflasters nach Anspruch 5, dadurch gekennzeichnet, daß das Verschweißen der Membran (120) und des dritten Pflasterteiles (150) einerseits mit der Wirkstoffträger (110)-Verbundfolie (114) andererseits gleichzeitig durchgeführt wird.

**Claims**

1. Pharmaceutical adhesive plaster with two combined parts, namely a carrier (10 ; 110) of active ingredient, comprising a bonded film (14 ; 114) provided with a shallow depression (16 ; 116) to hold medicament, possibly (Fig. 4) with an inner, particularly film-like coating (114.1) of thermoplastics material and, annularly encircling the depression (16 ; 116), a marginal coating (28 ; 150.3) of pressure and/or skin heat-sensitive adhesive on the open side of the depression (16 ; 116), and a composite covering film (12 ; 112), possibly (Fig. 4) with an inner, particularly film-like, coating (112.2) of thermoplastics material connected to the adhesive coating (28 ; 150.3) and covering the depression (16 ; 116) in the active ingredient carrier (10 ; 110), characterised in that the depression (16 ; 116) in the adhesive ingredient carrier (10 ; 110) is sealed by means of a membrane (20 ; 120) which is permeable to the active ingredient and which is securely connected to the composite film (14 ; 114) of the active ingredient carrier (10 ; 110), and in that the composite covering film (12 ; 112) is hermetically connected to the composite film (14 ; 114) of the active ingredient carrier (10 ; 110) along a ring (30 ; 158) extending between the membrane (20 ; 120) and the adhesive coating (28 ; 150.3).

2. Pharmaceutical adhesive plaster according to Claim 1, characterised in that the medicament (18 ; 118) containing or constituting the active ingredient and adapted to diffuse into the skin fills the depression (16 ; 116) in the active ingredient carrier (10 ; 110) by itself.

3. Pharmaceutical adhesive plaster according to Claim 1 or 2, characterised in that the bonded covering film (112) has its plane inside face bearing indirectly on the adhesive coating (150.3), this latter belonging to a third part (15) annularly surrounding the depression (115) and connected securely on the one hand to the bonded film (114) of the active ingredient carrier (110) and on the other to the bonded covering film (112) and being separable into two parts (150.1, 150.2 ; 150.3, 150.4) on the adhesive face (160) of the adhesive coating (150.3) which is towards the bonded covering film (112), such separation exposing this adhesive surface.

4. Pharmaceutical adhesive plaster according to one of Claims 1 to 3, characterised in that the membrane (120), like the third plaster part (150), consists of at least three layers (120.1 to 120.4), of which one, serving as an adhesive layer (120.3), is disposed between the other two layers (120.2. 120.4), and in that the membrane (120), possibly together with the third plaster part (150), is formed fron one piece.

5. Pharmaceutical adhesive plaster according to Claim 4, characterised in that the membrane (120) and the third plaster part (150) each consist of a thermoplastics layer (120.4 or 150.4) welded to a thermoplastics layer (114.1) of the bonded film (114) of the active ingredient carrier (110), also comprising the adhesive layer (120.3 or 150.3) applied to this layer (120.4 or 150.4), a thermoplastics layer (120.1 or 150.1) welded to a thermoplastics layer (112.2) of the bonded covering film (112) and of a layer (120.2 or 150.2), preferably of silicone, which is applied to this layer (120.1 or 150.1), this layer (120.2 or 150.2) bearing on the adhesive layer (120.3 or 150.3), the said adhesive layer (120.3 or 150.3) and also the synthetic plastics layer (120.4) of the membrane (120) which is connected to the bonded film (114) of the active ingredient carrier being permeable to the active ingredient, possibly the silicone layer (120.2) and/or the synthetic plastics layer (120.1) of the membrane (120) which is connected to the bonded covering film (112) not being permeable to the active ingredient.

6. Pharmaceutical adhesive plaster according to one of Claims 1 to 5, characterised in that the active ingredient carrier (110) bonded film (114) has, encircling its depression (116) and located outside the membrane (12) and inside the third plaster part (150), a raised portion (158) and/or the bonded covering film (112) comprises a corresponding raised portion, the height of which individually or jointly corresponds to the coincident thickness of the third plaster part (150) and of the membrane (120).

7. Method of producing the pharmaceutical adhesive plaster according to Claim 3, in which two separately produced outer plaster parts (110, 112) are separably connected by means of an inner adhesive layer (150.3), characterised in that the adhesive layer (150.3) is produced as a constituent part of an inner, third plaster part (150), which is produced separately from the outer two plaster parts (110 and 112) and is securely connected to them afterwards.

8. Method according to Claim 7 for producing the pharmaceutical adhesive plaster according to Claim 4, characterised in that the membrane (120) and the third plaster part (150) are produced simultaneously.

9. Method according to Claim 9 for producing the pharmaceutical adhesive plaster according to Claim 6, characterised in that the membrane (120) and the third plaster part (150) are produced in a single piece from which a ring corresponding to the raised portion (158) on the active ingredient carrier (110)-bonded film (114) is separated, singling out the membrane (120) and the third plaster part (150).

10. Method according to one of Claims 7 to 9 for producing the pharmaceutical adhesive plaster according to Claim 6 and 5, characterised in that the welding of the membrane (12), of the raised portion (158) of the active ingredient carrier (110)-bonded film (114) and of the third plaster part (150) on the one hand is carried out simultaneously with the bonded covering film (112) on the other.

11. Method according to one of Claims 7 to 9 or particularly Claim 10 for producing the pharmaceutical adhesive plaster according to Claim 5, characterised in that welding of the membrane (120) and of the third plaster part (150) on the one hand is carried out simultaneously with the active ingredient carrier (110)-bonded film (114) on the other.

**Revendications**

1. Pansement adhésif pharmaceutique comprenant deux parties réunies, à savoir un support de principe actif (10 ; 110), lequel comporte une feuille stratifiée (14 ; 114) présentant un évidement peu profond (16 ; 116) pour recevoir un médicament et éventuellement (figure 4) munie d'une couche intérieure (114.1), notamment en forme de feuille, en matière synthétique thermoplastique et, du côté de l'ouverture de l'évidement (16 ; 116), une couche marginale (28 ; 150.3), qui, composée d'une matière adhésive sensible à la pression et/ou à la chaleur de la peau, entoure l'évidement (16 ; 116) sous la forme d'une bague ; et une feuille stratifiée de recouvrement (12 ; 112), éventuellement (figure 4) munie d'une couche intérieure (112.2), en particulier en forme de feuille, en matière synthétique thermoplastique, qui est reliée à la couche de matière adhésive (28 ; 150.3) et recouvre l'évidement (16 ; 116) du support de principe actif (10 ; 110), caractérisé en ce que l'évidement (16 ; 116) du support de principe actif (10 ; 110) est fermé au moyen d'une membrane (20 ; 120) perméable au principe actif et reliée de manière fixe à la feuille stratifiée (14 ; 114) du support de principe actif (10 ; 110), et en ce que la feuille stratifiée de recouvrement (12 ; 112) est reliée hermétiquement à la feuille stratifiée (14 ; 114) du support de principe actif (10 ; 110) le long d'un anneau (30 ; 158) s'étendant entre la membrane (20 ; 120) et la couche de matière adhésive (28 ; 150.3).

2. Pansement adhésif pharmaceutique selon la revendication 1, caractérisé en ce que le médicament (18 ; 118) contenant ou constituant le principe actif et le faisant diffuser dans la peau remplit à lui seul l'évidement (16 ; 116) du support de principe actif (10 ; 110).

3. Pansement adhésif pharmaceutique selon la revendication 1 ou 2, caractérisé en ce que la feuille stratifiée de recouvrement (112) prend appui indirectement par l'intermédiaire de sa face intérieure plane sur la couche de matière adhésive (150.3), cette dernière appartenant à une troisième partie (150) qui, entourant l'évidement (116) en forme de bague, est reliée de manière fixe, d'une part, à la feuille stratifiée (114) du support de principe actif (110) et, d'autre part, à la feuille stratifiée de recouvrement (112) et peut, au niveau de celle (160) des surfaces d'adhérence de la couche de matière adhésive (150.3) qui est située du côté de cette feuille (112), être séparée en deux parties (150.1, 150.2 ; 150.3, 150.4), dont la séparation met cette surface d'adhérence à nu.

4. Pansement adhésif pharmaceutique selon l'une quelconque des revendications précédentes, caractérisé en ce que la membrane (120) de même que la troisième partie (150) du pansement est constituée d'au moins trois couches (120.1 à 120.4), l'une desquelles est disposée en tant que couche de matière adhésive (120.3) entre les deux autres couches (120.2, 120.4), et en ce que la membrane (120), éventuellement conjointement avec la troisième partie (150) du pansement, est réalisée en une seule pièce.

5. Pansement adhésif pharmaceutique selon la revendication 4, caractérisé en ce que la membrane (120) et la troisième partie (150) du pansement sont constituées respectivement d'une couche de matière synthétique thermoplastique (120.4 ; 150.4) soudée à une couche de matière synthétique thermoplastique (114.1) de la feuille stratifiée (114) du support de principe actif (110), de la couche de matière adhésive (120.3 ; 150.3) appliquée sur cette couche (120.4 ; 150.4), d'une couche de matière synthétique thermoplastique (120.1 ; 150.1) soudée à une couche de matière synthétique thermoplastique (112.2) de la feuille stratifiée de recouvrement (112), et d'une couche (120.1 ; 150.1), de préférence en silicone, appliquée sur la couche (120.1 ; 150.1) et qui est contiguë à la couche de matière adhésive (120.3 ; 150.3), la couche de matière adhésive (120.3 ; 150.3) ainsi que la couche de matière synthétique (120.4) constitutive de la membrane (120) et reliée à la feuille stratifiée (114) du support de principe actif étant perméables au principe actif et, éventuellement, la couche de silicone (120.2) et/ou la couche de matière synthétique (120.1) constitutive de la membrane (120) et reliée à la feuille stratifiée de recouvrement (112) étant imperméables au principe actif.

6. Pansement adhésif pharmaceutique selon l'une quelconque des revendications précédentes, caractérisé en ce que la feuille stratifiée (114) du support de principe actif (110) comporte une élévation (158) entourant l'évidement (116) de la feuille stratifiée (114) et située à l'extérieur de la membrane (120) et à l'intérieur de la troisième partie (150) du pansement et/ou la feuille stratifiée de recouvrement (112) comporte une élévation correspondante dont la hauteur, individuellement ou ensemble, correspond à l'épaisseur concordante de la troisième partie (150) du pansement et de la membrane (120).

7. Procédé pour la fabrication du pansement adhésif pharmaceutique selon la revendication 3, dans lequel deux parties extérieures (110 et 112), produites séparément, du pansement sont reliées au moyen d'une couche de matière adhésive intérieure (150.3) de manière à pouvoir être séparées l'une de l'autre, caractérisé en ce que la couche de matière adhésive (150.3) est produite en tant qu'élément constitutif d'une troisième partie intérieure (150) du pansement, laquelle est produite séparément des deux parties extérieures (110 et 112) du pansement et est ensuite reliée de manière fixe à ces dernières.

8. Procédé selon la revendication 7, pour fabriquer le pansement adhésif pharmaceutique de la revendication 4, caractérisé en ce que la membrane (120) et la troisième partie (150) du pansement sont réalisées simultanément.

9. Procédé selon la revendication 8, pour fabriquer le pansement adhésif pharmaceutique selon la revendication 6, caractérisé en ce que la membrane (120) et la troisième partie (150) du pansement sont réalisées en une seule pièce d'où est extrait un anneau correspondant à l'élévation (158) de la feuille stratifiée (114) du support de principe actif (110) et qui individualise la membrane (120) et la troisième partie (150) du pansement.

10. Procédé selon l'une quelconque des revendications 7 à 9, pour fabriquer le pansement adhésif pharmaceutique selon les revendications 5 et 6, caractérisé en ce que le soudage de la membrane (120), de l'élévation (158) de la feuille stratifiée (114) du support de principe actif (110), et de la troisième partie (150) du pansement, d'une part, à la feuille stratifiée de recouvrement (112), d'autre part, est effectué simultanément.

11. Procédé selon l'une quelconque des revendications 7 à 9, ou en particulier la revendication 10, pour fabriquer le pansement adhésif pharmaceutique selon la revendication 5, caractérisé en ce que le soudage de la membrane (120) et de la troisième partie (150) du pansement, d'une part, à la feuille stratifiée (114) du support de principe actif (110), d'autre part, est effectué simultanément.

Fig.1

Fig.2

Fig.3

Fig.4

0 171 800